# EUROPEAN PATENT SPECIFICATION

(11) **EP 2 415 487 B1**
(45) Date of publication and mention of the grant of the patent: **08.10.2014**
(21) Application number: 10171661.1
(22) Date of filing: 02.08.2010
(51) Int. Cl.: A61K 49/22, A61B 8/00, A61K 9/06, A61K 9/00, A61K 47/36

(54) **An ultrasonic couplant composition**
Ultraschallkopplungsmittelzusammensetzung
Composition de couplage ultrasonique

(43) Date of publication of application: 08.02.2012
(73) Proprietor: angioclinic AG, 8500 Frauenfeld (CH)
(72) Inventor: Ragg, Johann Christof, 10789 Berlin (DE)
(74) Representative: Kilger, Christian

(56) References cited:
- WO-A2-2004/103183
- WO-A2-2011/088333
- US-A1- 2006 246 111
- Budavari S. et al.: "The Merck Index", 1 January 1996 (1996-01-01), Merck Research Laboratories, Whitehouse Station, NJ * pages THER-3, THER-15, THER-16. *

## Description

### Field of the invention

The present invention relates to the field of pharmaceutical compositions. Particularly, the present invention relates to novel ultrasonic couplant composition for use in medical procedures requiring ultrasound-guided pen markings and punctures.

### Background of the invention

Diagnostic sonography (ultrasonography) is an ultrasound-based diagnostic imaging technique used to visualize subcutaneous body structures including fatty/connective tissue, tendons, muscles, joints, vessels and internal organs for possible pathology or lesions. Obstetric sonography is commonly used during pregnancy and is widely recognized by the public. Emergency ultrasound scans have become a well-known standard. A wide spectrum of diseases is diagnosed primarily by ultrasound, such as renal concrements or cysts, thyroid gland adenoma or cysts, liver lesions, hematoma, or venous insuffiency. Ultrasound has replaced more invasive methods like phlebography in the diagnosis or venous thrombosis or arteriography in the diagnosis of arterial obstructions. Often it is possible to perform both diagnosis and therapy in one session: Ultrasound can be used to guide interventional procedures, like tissue biopsies or collecting body fluid samples, positioning of drainage catheters, recanalizing instruments for occluded arteries or thrombosed veins, implantation of liver vein stents, or guiding other invasive tools. Due to the ultrasound guidance, a precise and 3-dimensional access to organs or lesions is obtainable, lowering side effects or complications in comparison to attempts using fluoroscopy (2-dimensional), or even no imaging. Ultrasound - guided interventions do not require x-ray like CT (computer tomography), and the patient is much better accessible than in the narrow tube of MRT (magnetic resonance tomography) which furthermore requires specialized nonmagnetic tools. Therefore, ultrasound - guided procedures are facing growing and important tasks in future.

Sonographers typically use a hand-held probe (called a transducer) that is placed directly on the the skin and moved over the patient. Since air will block the transmission of ultrasound signals, contact media between skin and transducer are required to obtain clear images. The contact media, or couplants, furthermore facilitate the movement of the transducer. The major demanded property of ultrasound couplants is to pass ultrasound signals without relevant loss, like water does, and to let the transducer slide over the skin.

When performing ultrasound-guided punctures or interventions, many problems arise which make it desirable to provide a ultrasonic couplant with enhanced properties and features::
Colour markings on patient's skin are common to support surgical or interventional treatments.
In interventional phlebology extended markings of the venous anatomy including healthy and diseased vessels are required: The markings indicate ultrasound findings of non-visible structures on the skin as an orientation for the surgical or interventional treatment. Usually, markings are performed when the patient is standing because venous disease (insufficiency) depends on the effect of gravitation. In horizontal positioning, like standard for vascular surgery or interventions, most of the pathology will not be detectable. The marking is decisive for the quality of the intervention. It may take longer than 30 minutes. The procedure is a relevant stress for patients and physicians. Faintings may occur in patients with low blood pressure. Therefore, all means to obtain a rapid and reliable marking are welcome.

Ultrasonic couplants of prior art, for example, often contain hydroxethylcellulose, a gelling and thickening agent derived from cellulose. US 2006246111 for example discloses ultrasound couplants comprising thickening polysaccharides, including starch. When using these ultrasonic couplants marking of the skin is not possible or at least laborious to mark target structures on the skin. The gelling and thickening agent of couplants of prior art ensheaths the marking device, e.g. a pen, and make it impossible to mark with said marking device. Thus, when using ultrasonic couplants of prior art the attending physician has to remove the couplant carefully to mark an indentified target structure (e.g. diseased vessels) on the skin of the subject. Thereafter further ultrasonic couplant has to be applied in order to continue diagnosis and/or therapy. Unfortunately, all known ultrasonic couplants interfere with the intention of colour markings on human skin. Ultrasonic couplants of prior art require time-consuming wiping and cleaning or even the use of cleaning fluids like alcohols to re-establish a markable skin surface. As ultrasound diagnostics and markings go step-by-step, the necessity of repeated cleaning, marking and new gel application is awful.

Furthermore, disinfectants, cleaning fluids or the ultrasound gel itself may ruin colour markings.

Thus, there is need of ultrasonic couplant composition allowing to mark target structures on the skin.

Furthermore, it is desirable to disinfect the area of the skin where the intervention will take place in order to avoid infections by puncturing. Nowadays the area is disinfected with commercially available compositions (e.g. Braunol). However, the skin has to be incubated with these compositions and applying the compositions on the skin before the ultrasonic couplant interferes with the transmission properties of the couplant.

As all interventional procedures require punctures at or near the side the sonography takes place. As punctures are associated with pain and discomfort of the patient. Thus, it is desirable to anesthetize the area the sonography and the punctuation shall take place. Commercially available anaesthetic ointments or sprays are not administrable as any effects will not show except occlusive bandages are applied for time-consuming intervals (1.5 - 30 min or even longer). Substances developing relevant pain relief to punctures within just a few minutes after application on the skin have not been reported so far. Further to a rapid analgesia an additional long-lasting anaesthetic effect is desirable to facilitate pain-free ambulation.

Thus, there is a need for an ultrasonic couplant compositions permitting diagnostic sonography, marking of the skin without time-consuming cleaning, disinfection, and local (skin) analgesia. By providing a ultrasonic couplant composition having all the desirable properties the present invention tremendously facilitates sonography and related interventions.

### Description of the invention

The above outlined problems are solved by the present invention through the provision of an ultrasonic couplant composition comprising:
An ultrasonic couplant composition comprising;
   (i) starch;
   (ii) a fast-acting local anaesthetic compound or a pharmaceutical acceptable salt thereof;
   (iii) a local anaesthetic compound with long lasting anaesthetic effect or a pharmaceutical acceptable salt thereof;
   (iv) an antiseptic compound or a pharmaceutical acceptable salt thereof;
   wherein the fast-acting local anaesthetic compound is selected from the group of lidocaine, mepivacaine, prilocaine, articaine, etidocaine, benzocaine, and 2-chlor procaine and the local anaesthetic compound with long lasting anaesthetic effect is selected from the group of bupivacaine, ropivacaine, procaine, and tetracaine.

In context with the present invention "starch" (or amylum) is a carbohydrate consisting of glucose units joined together by glycosidic bonds. It consists of two types of molecules: the linear and helical amylose and the branched amylopectin. Depending on the source, starch generally comprises 20 to 25% amylose and 75 to 80% amylopectin. The skilled artisan is aware that starch may be provided coming from different sources and in different degrees of purity. Starch may be for example derived from different types of plants. Furthermore, the skilled artisan knows that starch may be modified in order to adapt the properties, e.g. thickening of the ultrasonic couplant composition. Thus, in a preferred embodiment of the present invention the starch is selected from the group comprising corn starch, rice starch, wheat starch, potato starch, cassava starch and modified starch. The person of ordinary skills in the art knows modification of starch. He is furthermore aware of the modifications suited to reach the desired properties. A modified food starch is a starch that has been chemically modified to allow the starch to function properly under conditions frequently encountered during processing or storage, such as high heat, high shear, low pH, freeze/thaw and cooling. The modified starches are E coded according to the International Numbering System for Food Additives (INS) (CODEX ALIMENTARIUS published in FNP 52 Add 9 (2001)). Thus, in a preferred embodiment of the present invention the modified starch is selected from the group consisting of acid-treated starch (1401), alkaline-treated starch (1402), bleached starch (1403), oxidized starch (1404), starches, enzyme-treated (1405), monostarch phosphate (1410), distarch phosphate (1412), phosphated distarch phosphate (1413), acetylated distarch phosphate (1414), starch acetate (1420), acetylated distarch adipate (1422), hydroxypropyl starch (1440), hydroxypropyl distarch phosphate (1442), hydroxypropyl distarch glycerol (1443), Starch sodium octenyl succinate (1450), and acetylated oxidized starch (1451) (the respective INS numbers are given in brackets). However, in a preferred embodiment the starch is corn starch. In a further embodiment mixtures of two or more of the above mentioned starches are comprised in the ultrasonic couplant composition according to the present invention.

The starch in context with the present invention serves as a thickening agent. The skilled artisan artisan knows that the degree of thickening, i.e. the viscosity of the ultrasonic couplant composition may be varied by varying the concentration of the thickening agent, i.e. the starch. He will for example increase the starch concentration of the composition in order to increase the viscosity, accordingly he will decrease the concentration if a composition of lower viscosity is desirable. However, in a preferred embodiment of the present invention starch of the ultrasonic couplant composition has a concentration of 1 % to 15 %, preferably 6 % to 10 %, more preferably 4 % to 8 %. In one embodiment the starch of the ultrasonic couplant composition has a concentration of 8%.

The ultrasonic couplant composition of the present invention comprises local anaesthetic compounds. A local anaesthetic compound is a drug that causes reversible local anaesthesia and a loss of nociception. When it is used on specific nerve pathways (nerve block), effects such as analgesia (loss of pain sensation) and paralysis (loss of muscle power) can be achieved.

Local anaesthetics belong to one of three classes: amino amide, amino ester and amino amide ester local anaesthetics. Synthetic local anaesthetics are structurally related to cocaine. Most of the local anaesthetics share common structural features. The comprise a lipophilic group and a hydrophilic group connected by a alkyl chain differing in length, wherein the alkyl chain is bond to the lipophilic group through an ester bond or an amide bond or an ester and amide bond. Accordingly the local anaesthetics are grouped into amide-type local anaesthetic drug, amino ester-type local anaesthetic drug, and amino amide ester-type local anaesthetic drug (http://www.deutscher-apotheker-verlag.de/uploads/tx_crondavtitel/datei-datei/9783804725027 _p.pdf).

Local anaesthetics vary in their pharmacological properties, i.e. their period of anaesthetic effect and/or the period between application of the compound and initiation of the anaesthesia. In context of the present invention it is desirable that the ultrasonic couplant composition provides provides both, a rapid initiation of anaesthesia as well as a long lasting anaesthetic effect. The problem is solved by the present invention by providing a ultrasonic couplant composition comprising a fast-acting anaesthetic compound and the local anaesthetic compound with long lasting anaesthetic effect.

In context of the present invention a fast-acting anaesthetic compound initiates local anaesthesia within 10 s to 1 h after application of the compound on the skin of a subject, preferably within 30 s to 30 min after application, more preferably within 1 min to 15 min after application.

In context of the present invention a local anaesthetic compound has a long lasting anaesthetic effect if the local anaesthesia lasts for 1 h to 24 h after application of the compound on the skin of a subject, preferably for 2 h to 15 h, more preferably 4 h to 6 h. Also preferred are anaesthetic compound having a long lasting anaesthetic effect which cause a local anaesthesia for 4 h to 5 h.

According to the invention the fast-acting anaesthtic compound is selected from the group comprising lidocaine, mepivacaine, prilocaine, articaine, etidocaine, benzocaine, and 2-chlor procaine and the local anaesthetic compound with long lasting anaesthetic effect is selected from the group consisting of bupivacaine, ropivacaine, procaine, and tetracaine.

The skilled artisan is furthermore able to determine the desired concentration of the fast-acting anaesthetic compound. For example a high concentration may be desirable if the ultrasonic couplant is used at sensitive parts of the body in order to increase the anaesthetic effect. However, in a preferred embodiment of the ultrasonic couplant composition the fast-acting anaesthetic compound has a concentration of 1 mg/g to 100 mg/g, preferably 2 mg/g to 50 mg/g, more preferably 3 mg/g to 20 mg/g, even more preferably 4 mg/g to 10 mg/g. In a further preferred embodiment of the ultrasonic couplant composition according to the present invention the local anaesthetic compound with long lasting anaesthetic effect has a concentration of 1 mg/g to 100 mg/g, preferably 2 mg/g to 50 mg/g, more preferably 3 mg/g to 20 mg/g, even more preferably 4 mg/g to 10 mg/g.

The inventors unexpectedly found that by applying a ultrasonic couplant composition according to the present invention target areas of the skin can be within few minutes, e.g. within 2 to 5 min

For the present invention, the term antiseptic is meant to include to the following: antimicrobial agents, antibiotics, chemotherapeutic agents, any antiseptics, antiviral agents, viricidal agents, bactericidal agents, antifungal agents, antiparasitic agents, and the like. The words antiseptic, disinfectant, and vermicide all connote an agent which can kill microbes or infectious pathogens upon contact and thousands of chemical compounds are known which have antiseptic properties (For some examples see Remingtons Pharmaceutical Sciences: 1985, 1990 and Harvey, 1985). In a preferred embodiment of the present invention the antiseptic compound is bactericidal and/or fungicidal and/or virucidal, preferably the antiseptic compound is bactericidal and fungicidal or bactericidal and virucidal or fungicidal and virucidal. However, it is especially preferred that the antiseptic compound is bactericidal, fungicidal and virucidal.

Furthermore, more than one antiseptic compound may be comprised in the ultrasonic couplant composition according to the present invention. In a preferred embodiment the ultrasonic couplant comprises at least two antiseptic compounds, preferably at least three antiseptic compounds, more preferably at least four antiseptic compounds are comprised in the ultrasonic couplant according to the present invention. The antiseptic compounds may be chosen independently from the antiseptic compounds as outlined herein.

The antiseptic compound includes iodine, an iodine complex, an iodine complex, chlorhexidine, chlorhexidine salts, or combinations thereof. Preferred iodine complexes may include iodophors, e.g., povidone-iodine USP. Preferred chlorhexidine salts may include, e.g., chlorhexidine digluconate and chlorhexidine diacetate. Other suitable antiseptic compounds may include parachlorometaxylenol (PCMX), triclosan, hexachlorophene, fatty acid monoesters of glycerin and propylene glycol such as glycerol monolaurate, glycerol monocaprylate, glycerol monocaprate, propylene glycol monolaurate, propylene glycol manocaprylate, propylene glycol moncaprate, phenols, surfactants, and polymers that include a (C₁₂-C₂₂) hydrophobe and a quaternary ammonium group, polyquaternary amines such as polyhexamethylene biguanide as polyhexamethylene biguanide as well as those described in U.S. Pat. Nos. 6,440,405, 5,408,022 and 5,084,096, quaternary ammonium silanes, silver, silver salts (such as silver chloride), silver oxide and silver sulfadiazine, methyl, ethyl, propyl and butyl parabens, octenidene, peroxides (e.g., hydrogen peroxide and benzoyl peroxide), as well as combinations thereof

Interestingly, it has been found by the inventors that quaternary ammonium compounds are especially well suited for the ultrasonic couplant according to the present invention. Thus, in a preferred embodiment the antiseptic compound is a quaternary ammonium compound or a pharmaceutically acceptable salt thereof, more preferably the antiseptic compound is selected from the group comprising octenidin hydrochloride, benzalkonium chloride, benzethonium chloride, methylbenzethonium chloride, cetalkonium chloride, cetylpyridinium chloride, cetrimonium, cetrimide, dofanium chloride, tetraethylammonium bromide, didecyldimethylammonium chloride and domiphen bromide, povidone iodine, elemental iodine, sodium iodide, potassium iodide, sodium hypochlorite, nonoxynol-9, polihexanide and chlorhexidine gluconate.

Polihexanide, further to its antiseptic properties, enhances the healing of woundings, e.g. as they may be caused by puncture procedures or other interventions. Moreover, it has been found by the inventors that polihexanide further enhances the antiseptic properties of the ultrasonic couplant composition according to the present invention. In a preferred embodiment of the present invention the ultrasonic couplant composition further comprises a compound enhancing wound healing. In a preferred embodiment of the present invention the compound enhancing wound healing is selected from the group consisting of dexpanthenol; pantothenic acid.

The phrase "pharmaceutically acceptable salt(s)", as used herein, means those salts of compounds of the invention that are safe and effective for topical use in mammals and that possess the desired biological activity. Pharmaceutically acceptable salts include salts of acidic or basic groups present in compounds of the invention. Pharmaceutically acceptable acid addition salts include, but are not limited to, hydrochloride, hydrobromide, hydroiodide, nitrate, sulfate, bisulfate, phosphate, acid phosphate, isonicotinate, acetate, lactate, salicylate, citrate, citrate, tartrate, pantothenate, bitartrate, ascorbate, succinate, maleate, gentisinate, fumarate, gluconate, glucuronate, saccharate, formate, benzoate, glutamate, methanesulfonate, ethanesulfonate, benzenesulfonate, p-toluenesulfanate and pamoate (i.e., 1,1'-methylene-bis-(2-hydroxy-3-naphthoate)) salts. Certain compounds of the invention can form pharmaceutically acceptable salts with various amino acids. Suitable base salts include, but are not limited to, aluminium, calcium, lithium, magnesium, potassium, sodium, zinc, ammonium and diethanolamine salts. For a review on pharmaceutically acceptable salts see BERGE ET AL., 66 J. PHARM. SCI. 1-19 (1977).

Starch is used for the ultrasonic couplant composition. Starch is easily biodegradable. This increases the danger of contamination of the composition with microorganisms like fungi and/or bacteria. This problem is solved by the provision of an ultrasonic couplant composition comprising at least one preservative. Thus, in a preferred embodiment the ultrasonic couplant further comprises preservative compounds. A preservative is a naturally occurring or synthetic substance that is added to products such as pharmaceuticals to prevent decomposition by microbial growth or by undesirable chemical changes. Suited preservatives are known by those of ordinary skill in the art. In a preferred embodiment the preservative compound is bactericidal and fungicidal. In further preferred embodiment of the present invention the preservative compound is selected from the group comprising of phenoxyethanol.

In yet a further embodiment the ultrasonic couplant composition according to the present invention comprises at least two preservative compounds, preferably at least three, more preferably at least four preservative compounds. If more than one preservative compound is comprised in the ultrasonic couplant composition, the preservative compounds may be chosen independently from the preservative compounds as outlined herein. It is also preferred that the preservative compound is selected from the group comprising antioxidants, bactericidal compounds, fungicidal compounds, potassium sorbate, sodium benzoate, sodium methyl paraben, citric acid.

The ultrasonic couplant composition according to the present invention comprises starch as a thickening agent. Starch is insoluble in aqueous solution but forms a hydrocolloid after heating. However, it has been found that ultrasonic couplant compositions comprising starch tend to form precipitates or inhomogenities, which has been explained by the process of retrogradation. retrogradation. The inventors unexpectedly found that the problem of precipitations or inhomogenities of starch gel may be solved by adding small amounts of glycerole, fatty substances and/or emulsifying agents and/or starch degrading enzymes to the composition. Thus, in a preferred embodiment the ultrasonic couplant further comprises at least one compound selected from the group comprising glycerole, fats, emulsifying agents, or starch degrading enzymes.

The fatty substances to be used in the composition according to the invention may include vegetal oils (such as almond, jojoba and avocado oil), mineral oils, oils of animal origin (such as lanoline), synthetic oil (esters such as isopropyl myristate, decyl oleate, isopropyl palmitate), silicone oils (such as cyclornethicone and dimethicone) and fluorinated oils. Fatty alcohols and acids and waxes (such as bee wax and rice wax) may also be used. In a preferred embodiment of the present invention the ultrasonic couplant composition comprises at least two fatty substances, preferably at least three fatty substances, more preferably at least four fatty substances. If more than one fatty substance is comprised in the ultrasonic couplant composition, the fatty substances may be chosen independently from the fatty substances as outlined herein.

The emulsifying agents to be used in the composition may include polyglycerol fatty acid esters, saccharose fatty acid esters, sorbitan fatty acid esters, ethoxylated sorbitan fatty acid esters, ethers of fatty alcohols and PEG, glycerol fatty acid ethers, alkyl sulfates, alkyl ether sulfates, alkyl phosphates, alkyl polyglucosides, dimethicone co-polyols. Examples of ethoxylated emulsifiers are steareth-2 and steareth-21, while examples of glucoside-based emulsifyers are cetearyl glucoside and arachidyl glucoside. In a preferred embodiment of the present invention the ultrasonic couplant composition comprises at least two emulsifying agents, preferably at least three emulsifying agents, more preferably at least four emulsifying agents. If more than one emulsifying agents is comprised in the ultrasonic couplant composition, the emulsifying agents may be chosen independently from the emulsifying agents as outlined herein.

Starch degrading enzymes are known by those of ordinary skills in the art. Starch degrading enzymes are enzymes that break down or hydrolyze starch. Examples of such enzymes are α -and β-amylases. α-amylases are found in plants and in animals. However, α-amylase in context with the present invention may be of different sources including plants animals. Furthermore, also α-amylases which have been recombinantly expressed in microorganisms, such as bacteria and fungi, are encompassed by the present invention. In a further embodiment of the present invention the starch degrading enzyme is a β-amylase and/or α -amylases, preferably an α-amylase. β-amylase cuts starch into maltose units. The skilled artisan knows how to purify α-amylases and/or β-amylase from natural sources, i.e. plants or animals. He furthermore knows methods for recombinantly expressing α-amylase and/or β-amylase in microorganism, e.g. *Escherichia coli*, by using for example expression vectors.

Also disclosed herein is a method for producing an ultrasonic couplant composition according to the present invention comprising the steps of:
(i) mixing an aqueous solution of an local anaesthetic compound with long lasting anaesthetic effect or a pharmaceutical acceptable salt thereof and an antiseptic compound or a pharmaceutical acceptable salt thereof with starch to receive an suspension;
(ii) mixing the suspension with an antiseptic compound;
(iii) heating the suspension to solubilise the starch forming a hydrocolloid;
(iv) cooling the hydrocolloid to receive a hydrogel.

Mixing to receive suspension means that the aqueous solution of the local anaesthetic compounds according to the present invention are mixed thoroughly to form an heterogeneous fluid containing solid particles (i.e. starch particles).

It will be understood by those of ordinary skills in the art that the starch of the suspension gets solubilised when heated forming a hydrocolloid.

A hydrocolloid is defined as a colloid system wherein the colloid particles are dispersed in water or an aqueous solution or buffer. A hydrocolloid has colloid particles spread throughout water or an aqueous solution or buffer, and depending on the quantity of water available and/or temperature that can take place in different states, e.g. gel (hydrogel) or sol (liquid). Hydrocolloids can be either irreversible (single-state) or reversible. Starch and aqueous solutions, for example, form a reversible hydrocolloid that can exist in a gel and sol state, and alternate between states with the addition or elimination of heat.

The skilled artisan can choose the temperature needed to solubilise the starch. He is aware of the fact that solubilisation may not only be influenced by the temperature the emulsion is heated to but also by the time/period for which the temperature is hold. In a preferred embodiment of the present invention the suspension is heated to at least 57°C to solubilise the starch, preferably to at least 60°C, more preferably to at least 80°C, further preferred to at least 95°C. In one embodiment of the invention the suspension is heated to boiling of the emulsion. However, it will be understood by those skilled in the art that it might be desirable to prevent the suspension from boiling in order to avoid the formation of small bubbles in the couplant that may interfere with the ultrasonic transmission. Thus, in a especially preferred embodiment of the present invention the suspension is heated to 95°C to solubilise the starch. In a yet further preferred embodiment the temperature to solubilise the starch is hold for 1 s to 5 min, preferably 5 s to 2 min, more preferably 10 s to 1 min, even more preferably 15 s to 30 s. In a yet further preferred embodiment emulsion is boiled for 5 s to 5 min to solubilise the starch, preferably 5 s to 2 min, more preferably 10 s to 1 min, even more preferably 15 s to 30 s. Preferably the suspension is heated to 80°C to 95°C for 15 s to solubilise the starch, preferably to 85°C to 95°C for 15s.

In a further embodiment the suspension is continuously mixed (e.g. by stirring) during heating. In a further preferred embodiment of the present invention the suspension, hydrocolloid and hydrogel is mixed throughout all steps of the method according to the present invention.

After solubilising the starch it is desirable to transform the hydrocolloid from the sol state to a gel state. Therefore, the hydrocolloid is cooled down to a temperature at which a hydrogel is obtained. In a preferred embodiment of the method according to the present invention in step (iv) of the method the hydrocolloid is cooled to a temperature between 4°C and 70°C to obtain a hydrogel, preferably between 10°C and 50°C, more preferably between 20°C and 45°C . In a preferred embodiment the hydrocolloid is cooled to room temperature to obtain a hydrogel. In a further preferred embodiment the hydrocolloid is cooled to a temperature between 1°C to 7°C. Furthermore, ultrasonic couplant composition obtainable by the method according to the present invention may be stored at 1°C to 7°C. However, storage may also occur at room temperature, as the comprised compounds preserve the composition from decomposition, e.g. by by microorganisms.

It will be readily understood that cooling of the hydrocolloid may occur actively, i.e. by using cooling devices, or it may occur passively by incubation of the mixture at room temperature. In a preferred embodiment of the present invention the hydrocolloid is cooled by using a cooling device, wherein the cooling rate is controllable.

It has been unexpectedly found by the inventors of the present invention that the cooling rate may have a great influence on the consistency/texture of the resulting hydrogel. Thus, in a preferred embodiment of the present invention the cooling rate of the hydrocolloid is controlled. In a further preferred embodiment the hydrocolloid is cooled at a rate of 1°C/h to 60°C/h, preferably 5°C/h to 40°C/h, more preferably 10°C/h to 20°C/h. In an especially preferred embodiment of the method according to the present invention the hydrocolloid is cooled at a rate of 15°C/h.

As outlined above in great detail, the ultrasonic couplant composition according to the present invention may comprise fats and/or emulsifying agents and/or starch degrading enzymes. It will be readily understood by those of ordinary skills that these components may be added at different steps of the method according to the present invention. However, if the component is not stable at high temperatures, e.g. the temperature used for solubilising the starch, it may be desirable to add and mix the component after the heating of the emulsion. Thus, in one embodiment the method according to the present invention comprises as a further step (v) mixing the hydrogel with a compound selected from the group comprising fatty substances, emulsifying agents, and starch degrading enzymes. However, in a further embodiment a compound selected from the group comprising fatty substances, emulsifying agents, and starch degrading enzymes is added and mixed to the emulsion before heating to solubilise the starch.

Furthermore it will be understood by those of ordinary skills that the emulsion may be preheated before mixing with the antiseptic compound. In a preferred embodiment of the present invention the emulsion is preheated to a temperature of 30°C to 90°C before step (ii) of the method according to the present invention, preferably to a temperature of 40°C to 80°C, more preferably to a temperature of 60°C to 80°. Furthermore, in one embodiment of the present invention a preservative according to the present invention is added and mixed to the emulsion in step (ii) of the present invention.

The inventors unexpectedly found that the ultrasonic couplant composition according to the present invention allows direct marking of target structures on the skin without removing the couplant. By this property the present invention provides a ultrasonic couplant composition facilitating sonography assisted interventions. Moreover the inventors unexpectedly found that the skin may be marked through the application of iodine comprising agents on the skin covered with the ultrasonic couplant according to the present invention. For example the iodine comprising agents, preferably a liquid, may be applied to the skin with a cotton swap soaked with the agent. Unexpectedly the markings are not influenced by applying the ultrasound and remains at their position. However, after the intervention the markings can be removed by using alcohol, e.g. ethanol and/or isopropanol, and/or solution comprising antioxidants (e.g. vitamine C).

Disclosed is also a device comprising:
a) a cone-shaped tip connectable to "Luer"-tapper syringes;
b) a cone-shaped solid adsorptive tissue positively fitted into the cone-shaped tip;
c) optionally a cap to cover the opening of the cone-shaped tip;
wherein the plastic tip comprises an opening opposite of the side connectable to the "Luer" tapper syringe and an opening on the side connectable to the "Luer" tapper syringe to connect the cone-shaped tip with the syringe.

The "Luer"-tapper system and syringes are known by those skilled in the art and are commercially available ( B.Braun Melsungen, Germany).

It will be acknowledged by those skilled in the art that the device may be connected to a "Luer"-tapper syringe. Said syringe may be filled with a marking agent, e.g. a fluid comprising iodide and/or potassium iodide. Thereby, the marking agent may be applied to the solid adsorptive tissue and by increasing the pressure, e.g. by using the piston of the syringe, the marking agent may be applied to the skin through the opening of opposite of the side connectable to the "Luer"-tapper syringe.

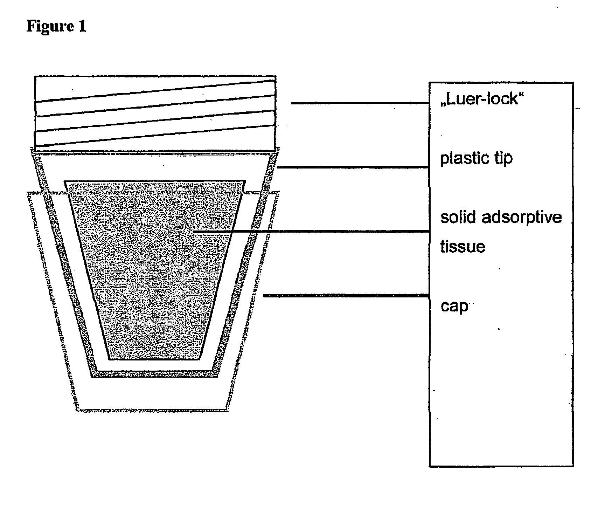

The device is exemplified in Figure 1.

Furthermore, it has been unexpectedly found be the inventors that the ultrasonic couplant composition provides further unexpected properties. Since the ultrasonic couplant composition according to the present invention does not contain substances aggressive to the transducer membrane (e.g. ethanol, isopropyl alcohol) the transducer may be directly applied to the couplant without the use of a cover (e.g. a membrane). As the ultrasonic couplant composition according to the present invention is antiseptic, the risk of microbial contamination of the transducer is averted. This furthermore allows the waiver of a cover. However, covers may be used to simplify hygienic cleaning. Such covers are known by those of ordinary skills in the art.

Disclosed is also a kit comprising an ultrasonic couplant composition according to the present invention.

As used herein, a kit is a packaged combination optionally including instructions for use of the combination and/or other reactions and components for such use.

The kit may further comprise a device and/or substance for marking the skin. In a further embodiment of the present invention the device for marking the skin of a subject comprises iodine. Devices and substances for marking the skin of a subject are known by those skilled in the art. They may for example be selected from the group consisting of marker pens, swabs and marking liquids comprising iodine. Liquids comprising iodine are also known in the art and may e.g. be selected from the group comprising Braunol®.

Disclosed is also a ultrasonic couplant composition according the present invention or a kit according the present invention for use in sonography or sonography assisted interventions, e.g. the sonography is performed to catheterize venes and/or arteries and/or other and/or other organs, to diagnose obstruction of arteries, inner organs or other tissue, to diagnose deep vein thrombosis (DVT) (Thrombosonography), and/or to determine extent and severity of venous insufficiency (venosonography)

### Examples

### Example 1: Ultrasound transmission:

The ultrasonic couplant composition according to the present invention provides excellent ultrasound transmission, in particular when performing ultrasound-guided punctures. The ultrasonic couplant composition is compared to a ultrasonic couplant of prior art (Aquasonic®). The two ultrasonic couplants were applied to the skin of patients to scan various tissue structures (skin, fat, fasciae, muscles, blood vessels) with longitudinal transducers (7,5 - 14 MHz) by various investigators with different levels of experience. in no case visual differences in 128/128 high-resolution screen samples are observed between the optimised ultrasonic couplant of prior art and the ultrasonic couplant composition according to the present invention.

### Example 2: Disinfection

As outlined above, the ultrasonic couplant composition according to the present invention has antiseptic properties. Thus, the need for additional disinfectants is lapsed.

The antiseptic properties of the ultrasonic couplant composition according to the present invention is compared with common German/intemational standards of surgical disinfectants (Octenicid, Octeniderm, Braunol), and sterile samples of the standard ultrasound couplant "Aquasonic" as control: 2 g of the substance or composition are applied to 20 skin areas of 10 x 10 cm each. The substances or composition are spread with an ultrasound transducer for 10 seconds. Then the gel is wiped off with one single passage of Kleenex tissue. Samples are taken of each area by contacting a nutrient agar plate with the skin for 3 seconds. The evaluation is performed by the Institute for Mikrobiology of Lichtenberg Hospital Berlin. Results are shown in table 1.

**Table 1: Cultivated bacterial colonies**

| **Substance/compositions** | **Cultivated bacterial colonies on the aga plate** |
|---|---|
| Ultrasonic couplant composition according the present invention | 0-1 |
| Octenicid | 2-8 |
| Octeniderm | 2-8 |
| Braunol | 2-8 |
| Aquasonic | >200 |

This clearly shows that the ultrasonic couplant composition unexpectedly provides a tremendously increased (2 to 8 time) antiseptic effect than standard disinfectants.

Therefore, no additional or precedent disinfectant is required in the area of interventional (or surgical) procedures when using the ultrasonic couplant composition according to the present invention. This further avoids the pollution of air by the spray disinfectants.

### Example 3: Analgesia

The analgetic properties of the ultrasonic couplant composition according to the present invention are compared with a starch hydrogel containing only water and starch as negative controland an European standard ointment for skin anaesthesia (EMLA, 1 g containing 25 mg Lidocaine and 25 mg Prilocaine), in a randomized and double-blinded study, 2 grams of each substance are applied on neighbouring areas and covered with plastic film for 20 min.

Three adjacent areas of 3 x 3 cm are marked on different body parts of 20 patients and 20 (ventral/dorsal side of thigh, lower leg; arms, belly). In every test area 5 similar locations were marked resembling the "5" of dice. To avoid saturation of sensitivity, all subjects have only two body regions included. The allocation of substance and test area is randomized and double-blinded. Successive triples of punctures (30G needle, B. Braun, Melsungen, Germany) are performed, randomized in sequence one in each test area in five series. After each triple of punctures the pain was rated by the subject: The most painful puncture received 0 points, intermediate 1, and least pain 2 points. If undetermined, 0 points were given for "pain", 1 for "discomfort" and 2 for "no pain". The result showed 585 points for the ultrasonic couplant composition according to the present invention, 180 for the negative control, and 375 for EMLA.

This shows that the ultrasonic couplant composition according to the present invention, further to the other unexpected properties, has a tremendously enhanced an analgetic effect, even higher than the effect of standard analgetic substances.

Also the period between application of the ultrasonic couplant composition and the appearance of the anaesthetic effect is shorter than for EMLA: testing the ultrasonic couplant composition according to the present invention in 20 subjects changes in local sensitivity could be felt already within 2-3 minutes by the majority of the subjects, even when abandoning occlusive films (8/10 subjects), while EMLA took at least 15 minutes to show an effect, and often failed totally (6/10 subjects) when no occlusive film was used.

These results clearly show that the properties of the ultrasonic couplant composition according to the present invention qualify the composition to perform enhanced sonography assisted interventions. The (micro) punctures in interventional phlebology (e.g. local anaesthesia, vascular access) or cosmetic vascular treatments (e.g. spider veins) can be performed an at significant lower pain level, superior to all other known ultrasonic couplant compositions.

### Example 4: Marking of the skin

The inventors found by incidence, that disinfectant fluids containing iodine will react with the new gel to a brownish colour. When potassium iodide is added, due to the known LUGOL reaction blue to violet colours can be produced. When using a gel film of 0,1 - 1 mm thickness, markings will reach the skin and stay there in spite of further ultrasound activity, or wiping.

Puncture sites or anatomic landmarks can be marked by this method directly on the gel, without any need for cleaning. Initially performed markings of superficial structures like varicose veins using water-resistant felt-pens will not be spoiled by the gel. All markings remain correctable and easily removable after conclusion of the treatment by alcoholic solutions or solutions of Vitamin C (antioxydants).

As an advantage, diluted iodide/potassium iodide solutions are an agent with no potential toxicity or allergy provocation.

## Claims

1. An ultrasonic couplant composition comprising;
(i) starch;
(ii) a fast-acting local anaesthetic compound or a pharmaceutical acceptable salt thereof;
(iii) a local anaesthetic compound with long lasting anaesthetic effect or a pharmaceutical acceptable salt thereof;
(iv) an antiseptic compound or a pharmaceutical acceptable salt thereof;
wherein the fast-acting local anaesthetic compound is selected from the group of lidocaine, mepivacaine, prilocaine, articaine, etidocaine, benzocaine, and 2-chlor procaine and the local anaesthetic compound with long lasting anaesthetic effect is selected from the group of bupivacaine, ropivacaine, procaine, and tetracaine.

2. The ultrasonic couplant composition according to claim 1, wherein the starch is selected from the group comprising corn starch, rice starch, wheat starch, potato starch, cassava starch and modified starch.

3. The ultrasonic couplant composition according to claim 1 or 2, wherein the starch has a concentration of 2 % to 20 %, preferably 4 % to 15 %, more preferably 6 % to 10 %.

4. The ultrasonic couplant composition according to any one of claims 1 to 3, wherein the fast-acting anaesthetic compound has a concentration of 1 mg/g to 100 mg/g, preferably 2 mg/g to 50 mg/g, more preferably 3 mg/g to 20 mg/g, even more preferably 4 mg/g to 10 mg/g.

5. The ultrasonic couplant composition according to any one of claims 1 to 4, wherein the local anaesthetic compound with long lasting anaesthetic effect has a concentration of 1 mg/g to 100 mg/g, preferably 2 mg/g to 50 mg/g, more preferably 3 mg/g to 20 mg/g, even more preferably 4 mg/g to 10 mg/g.

6. The ultrasonic couplant composition according to any one of claims 1 to 5, wherein the antiseptic compound is bactericidal, fungicidal and virucidal.

7. The ultrasonic couplant according to claim 6, wherein the antiseptic compound is a quaternary ammonium compound.

8. The ultrasonic couplant composition according to any one of claims 1 to 7, wherein the antiseptic compound is octenidin hydrochloride, benzalkonium chloride, benzethonium chloride, methylbenzethonium chloride, cetalkonium chloride, cetylpyridinium chloride, cetrimonium, cetrimide, dofanium chloride, tetraethylammonium bromide, didecyldimethylammonium chloride and domiphen bromide.

9. The ultrasonic couplant composition according to any one of claims 1 to 8, wherein the ultrasonic couplant further comprises at least one preservative compound, wherein the preservative compound is bactericidal and fungicidal.

10. The ultrasonic couplant composition according to claim 9, wherein the preservative compound is selected from the group comprising of phenoxyethanol.

11. The ultrasonic couplant composition according to any one of claims 1 to 10, wherein the ultrasonic couplant further comprises at least one compound selected from the group comprising fatty substances, emulsifying agents, starch degrading enzymes.

12. The ultrasonic couplant composition according to claim 11, wherein the starch degrading enzyme is an α-amylase.

## Patentansprüche

1. Ultraschallkopplungszusammensetzung, umfassend
(i) Stärke;
(ii) ein schnell wirksames Lokalanästhetikum oder ein pharmazeutisch akzeptables Salz davon
(iii) ein Lokalanästhetikum mit Langzeitwirkung oder ein pharmazeutisch akzeptables Salz davon
(iv) ein antiseptischer Wirkstoff oder ein pharmazeutisch akzeptables Salz,
wobei das schnell wirksame Lokalanästhetikum aus der Gruppe der folgenden Stoffe ausgewählt wird: Lidocain, Mepivacain, Prilocain, Articain, Etidocain, Benzocain und 2-Chlor-Procain und das Lokalanästhetikum mit Langzeitwirkung aus folgender Gruppe ausgewählt wird: Bupivacain, Ropivacain, Procain und Tetracain.

2. Die Ultraschallkopplungszusammensetzung nach Anspruch 1, wobei die Stärke aus der Gruppe umfassend Maisstärke, Reisstärke, Weizenstärke, Kartoffelstärke, Maniokstärke und modifizierte Stärke ausgewählt wird.

3. Die Ultraschallkopplungszusammensetzung nach einem der Ansprüche 1 und 2, wobei die Stärke eine Konzentration von 2-20 %, bevorzugt 4-15 % und mehr bevorzugt 6-10 % aufweist.

4. Die Ultraschallkopplungszusammensetzung nach einem der Ansprüche 1 bis 3, wobei das schnellwirksame Lokalanästhetikum eine Konzentration von 1-100 mg/g aufweist, bevorzugt 2-50 mg/g, mehr bevorzugt 3-20 mg/g, am bevorzugtesten 4-10 mg/g.

5. Die Ultraschallkopplungszusammensetzung nach einem der Ansprüche 1 bis 4, wobei das Lokalanästhetikum mit Langzeitwirkung eine Konzentration von 1-100 mg/g, bevorzugt 2-50 mg/g, mehr bevorzugt 3-20 mg/g, am bevorzugtesten 4-10 mg/g aufweist.

6. Die Ultraschallkopplungszusammensetzung nach einem der Ansprüche 1 bis 5, wobei die antiseptische Verbindung bakterizid, fungizid und viruzid ist.

7. Die Ultraschallkopplungszusammensetzung nach Anspruch 6, wobei die antiseptische Verbindung eine quaternäre Ammoniumverbindung ist.

8. Die Ultraschallkopplungszusammensetzung nach einem der Ansprüche 1 bis 7, wobei die antiseptische Substanz Octenidinhydrochlorid, Benzalkoniumchlorid, Benzethoniumchlorid, Methylbenzethoniumchlorid, Cetalkoniumchlorid, Cetylpyridiniumchlorid, Cetrimonium, Cetrimid, Dofaniumchlorid, Tetraethylammoniumbromid, Didecyldimethylammoniumchlorid und Domiphenbromid ist.

9. Die Ultraschallkopplungszusammensetzung nach einem der Ansprüche 1 bis 8, wobei das Ultraschallkopplungsmittel weiterhin wenigstens eine weitere konservierende Substanz enthält, wobei die konservierende Substanz bakterizid und fungizid ist.

10. Die Ultraschallkopplungszusammensetzung nach Anspruch 9, wobei das Schutzmittel aus der Gruppe beinhaltend Phenoxyethanol ausgewählt wird.

11. Die Ultraschallkopplungszusammensetzung gemäß einem der Ansprüche 1 bis 10 ist, wobei das Ultraschallkopplungsmittel weiterhin wenigstens eine weitere Substanz ausgewählt aus der Gruppe umfassend Fette, Emulgatoren und stärkeabbauende Enzyme enthält.

12. Die Ultraschallkopplungszusammensetzung nach Anspruch 11, wobei das stärkeabbauende Enzym eine α-Amylase ist.

## Revendications

1. Composition de couplage ultrasonique comprenant :
(i) de l'amidon ;
(ii) un composé anesthésique local à action rapide ou un sel pharmaceutiquement acceptable de celui-ci ;
(iii) un composé anesthésique local ayant un effet anesthésique longue durée ou un sel pharmaceutiquement acceptable de celui-ci ;
(iv) un composé antiseptique ou un sel pharmaceutiquement acceptable de celui-ci ;
dans laquelle le composé anesthétique local à action rapide est sélectionné dans le groupe de la lidocaïne, la mépivacaïne, la prilocaïne, l'articaïne, l'étidocaïne, la benzocaïne et la 2-chloroprocaïne et le composé anesthésique local ayant un effet anesthésique longue durée est sélectionné dans le groupe de la bupivacaïne, la ropivacaïne, la procaïne, et la tétracaïne.

2. Composition de couplage ultrasonique selon la revendication 1, dans laquelle l'amidon est sélectionné dans le groupe comprenant l'amidon de maïs, l'amidon de riz, l'amidon de blé, l'amidon de pomme de terre, l'amidon de manioc et l'amidon modifié.

3. Composition de couplage ultrasonique selon la revendication 1 ou 2, dans laquelle l'amidon a une concentration de 2 % à 20 %, de préférence de 4 % à 15 %, de manière davantage préférée de 6 % à 10 %.

4. Composition de couplage ultrasonique selon l'une quelconque des revendications 1 à 3, dans laquelle le composé anesthésique à action rapide a une concentration de 1 mg/g à 100 mg/g, de préférence de 2 mg/g à 50 mg/g, de manière davantage préférée de 3 mg/g à 20 mg/g, de manière encore davantage préférée de 4 mg/g à 10 mg/g.

5. Composition de couplage ultrasonique selon l'une quelconque des revendications 1 à 4, dans laquelle le composé anesthésique local ayant un effet anesthésique longue durée a une concentration de 1 mg/g à 100 mg/g, de préférence 2 mg/g à 50 mg/g, de manière davantage préférée de 3 mg/g à 20 mg/g, de manière encore davantage préférée de 4 mg/g à 10 mg/g.

6. Composition de couplage ultrasonique selon l'une quelconque des revendications 1 à 5, dans laquelle le composé antiseptique est bactéricide, fongicide et virucide.

7. Couplant ultrasonique selon la revendication 6, dans lequel le composé antiseptique est un composé ammonium quaternaire.

8. Composition de couplage ultrasonique selon l'une quelconque des revendications 1 à 7, dans laquelle le composé antiseptique est le chlorhydrate d'octénidine, le chlorure de benzalkonium, le chlorure de benzéthonium, le chlorure de méthylbenzéthonium, le chlorure de cétalkonium, le chlorure de cétylpyridinium, le cétrimonium, le cétrimide, le chlorure de dofanium, le bromure de tétraéthylammonium, le chlorure de didécyldiméthylammonium et le bromure de domiphène.

9. Composition de couplage ultrasonique selon l'une quelconque des revendications 1 à 8, dans laquelle le couplant ultrasonique comprend en outre au moins un composé conservateur, dans lequel le conservateur est bactéricide et fongicide.

10. Composition de couplage ultrasonique selon la revendication 9, dans laquelle le composé conservateur est sélectionné dans le groupe comprenant le phénoxyéthanol.

11. Composition de couplage ultrasonique selon l'une quelconque des revendications 1 à 10, dans laquelle le couplant ultrasonique comprend en outre au moins un composé sélectionné dans le groupe comprenant les substances grasses, les agents émulsifiants, les enzymes de dégradation de l'amidon.

12. Composition de couplage ultrasonique selon la revendication 11, dans laquelle l'enzyme de dégradation de l'amidon est une α-amylase.
